# EUROPEAN PATENT APPLICATION

(11) **EP 4 026 841 A1**
(43) Date of publication of application: **13.07.2022**
(21) Application number: 20861589.8
(22) Date of filing: 03.09.2020
(51) Int. Cl.: C07K 5/083, C07K 5/097, C07K 5/10, C07K 7/06, C12N 5/071, C12N 5/0735, C12N 11/04, C12N 11/08

(54) **COMPOSITION CONTAINING THERMOSENSITIVE GEL AND OLIGOPEPTIDE, AND USE THEREOF**

(30) Priority: 06.09.2019 JP 2019163166
(71) Applicant: International Frontier Technology Laboratory Inc., Tokyo 105-0001 (JP)
(72) Inventor: KOMATSU Nobuaki, Tokyo 105-0001 (JP); WAKABAYASHI Machiko, Tokyo 105-0001 (JP); ITO Tomoko, Tokyo 105-0001 (JP)
(74) Representative: Bandpay & Greuter
(86) International application number: PCT/JP2020/033487
(87) International publication number: WO 2021/045166

(57) **Abstract**

The present disclosure relates to a composition which can promote cell proliferation, can be used for coating a wound or the like and culturing or preserving cells and tissues, and comprises a polymer compound. The composition of the present disclosure comprises a polymer compound which has a sol-gel transition temperature, reversibly transforms to a sol state at a temperature lower than the transition temperature, and comprises hydrophobic and hydrophilic portions and an oligopeptide having a cell proliferation function.

## Description

### Technical Field

The present disclosure relates to a composition comprising a sensitive gel and a peptide and use of the composition. More specifically, the present disclosure relates to a composition comprising a polymer compound (thermoreversible gelation polymer (TGP)) which has a sol-gel transition temperature and reversibly exhibits a liquid state (sol state) at a temperature lower than the sol-gel transition temperature and an oligopeptide having a cell proliferation function. The composition of the present disclosure can be used for a wound-coating agent, proliferation of various cells, preservation of living tissues, and the like.

### Background Art

Conventionally, in culturing a cell such as a cancer cell, a culture substrate using a gel matrix such as collagen, agar, or a polymer compound may be used.

For example, some polymer compounds have a sol-gel transition temperature and transform to a liquid state (sol state) at a temperature lower than the transition temperature and to a solid state (gel state) at a temperature higher than the transition temperature. Such polymer compounds are used as a scaffold for culturing a cancer cell and an epithelial cell (Japanese Patent No. 3190145: Patent Literature 1, Japanese Patent No. 3190147: Patent Literature 2).

Active substances having the function of promoting the proliferation of various cells are also known. In particular, a specific peptide is known as an active substance having the function of promoting the proliferation of an epithelial cell (Japanese Patent No. 4654418: Patent Literature 3).

A gauze, powdery agent, ointment, cream agent, and the like have been used as a coating agent for a wound. Most of them are intended to protect a wound surface which is an affected portion and enhance the effect of healing the affected portion.

Coating agents using a hydrocolloid and a hydrogel (Japanese Patent Laid-Open No. 2018-121756: Patent Literature 4), a laminate including a collagen film (Japanese Patent Laid-Open No. 2018-187850: Patent Literature 5), and the like are known in these days.

Some of these coating agents include an active substance having a sterilization function on the surface of a coating agent, a layer adjacent to the coating agent, or the like as required.

### Citation List

### Patent Literature

PTL 1: Japanese Patent No. 3190145
PTL 2: Japanese Patent No. 3190147
PTL 3: Japanese Patent No. 4654418
PTL 4: Japanese Patent Laid-Open No. 2018-121756
PTL 5: Japanese Patent Laid-Open No. 2018-187850

### Summary of Invention

### Technical Problem

Conventional coating agents can coat an affected portion including a scald (a socalled burn; hereinafter also referred to as a burn in the present specification) or a wound portion and enhance a treatment effect. However, in removing the coating agent from the burn or a wound surface (e.g., replacing the coating agent), in a case where the coating agent adheres to the burn or the wound surface, the burn or the wound surface which is healing may be damaged.

An object of the present disclosure is to provide a coating agent which adequately protects a burn and a wound, can promote healing of the burn and wound as required, and can be easily washed away.

An object of the present disclosure is to provide a method of treating a burn or a wound using the composition or the coating agent described above.

Further, an object of the present disclosure is to provide a culture substrate which enables efficient proliferation of a desired cultured cell and can simply and easily recover obtained cells.

An object of the present disclosure is to provide a substrate which can be used to preserve and transport a living tissue.

An object of the present disclosure is to provide a composition which can be used for the above coating agent, culture substrate, substrate for preserving and transporting a living tissue, and the like.

### Solution to Problem

The present disclosure relates to a composition comprising:
(A) a polymer compound which has a sol-gel transition temperature, reversibly transforms to a sol state at a temperature lower than the transition temperature, and comprises hydrophobic and hydrophilic portions; and
(B) an oligopeptide having a cell proliferation function.

The oligopeptide having the cell proliferation function is preferably a water-soluble oligopeptide having 3 to 7 amino acids comprising a prolyl-isoleucyl-glycyl unit or an isoleucyl-glycyl-serine unit, and a water-soluble salt thereof.

The cell proliferation function of the oligopeptide described above is preferably an epithelial cell proliferation function.

The above polymer compound preferably has a sol-gel transition temperature in a range of 20°C to 45°C.

The above composition preferably further comprises a bactericide, a local anesthetic, a pain reliever, and a preservative.

The present disclosure is directed to a coating agent, a cell-culturing substrate (e.g., a three-dimensional culture substrate for an iPS cell or an ES cell), and a living tissue-transporting substrate which comprise the above composition.

It is considered that the composition of the present disclosure can be used for culturing cells such as an epithelial cell, a cancer cell, an iPS cell, or an ES cell.

### Advantageous Effects of Invention

The composition of the present disclosure comprises a polymer compound capable of reversibly performing a sol-gel transition depending on an ambient temperature and an oligopeptide having a predetermined cell proliferation function, and thus can be used for a coating agent, a cell-culturing substrate, and preservation or transportation of a living tissue.

The coating agent in particular can be in a liquid state in an initial state and thus easily coats a burn or a wound surface and can transform to a solid state due to the temperature of a coated portion to coat the burn or wound portion. In the case of removing the coating agent, the coating agent on the coated portion changed to a liquid state by cooling the coated portion, so that the coating agent can be easily washed away and can be removed without damaging the coated portion.

Further, the present disclosure relates to a method of treating a burn or a wound. A treatment method of the present disclosure comprises:
step a) applying a composition or a coating agent comprising the composition to an affected portion comprising a burn or a wound, the composition comprising:
(A) a polymer compound which has a sol-gel transition temperature, reversibly transforms to a sol state at a temperature lower than the transition temperature, and comprises hydrophobic and hydrophilic portions; and
(B) an oligopeptide having a cell proliferation function,
   wherein the composition or the coating agent causes a sol-gel transition on the affected portion and transforms to a sol state thereby to coat the affected portion. The treatment method of the present disclosure can further comprise, after step a), cooling the composition or the coating agent to remove the composition or the coating agent from the affected portion.

In the treatment method of the present disclosure, the composition or the coating agent of the present disclosure coating the affected portion is cooled, so that the composition or the coating agent of the present disclosure transforms to a liquid state and can be easily washed away. This reduces a possibility that the burn and the wound surface which are healing may be damaged.

### Brief Description of Drawings

[FIG. 1] FIG. 1 is a photograph showing a state at the time of applying a composition of the present disclosure to an arm (corresponding to a substrate) and showing that the composition is in a sol state.
[FIG. 2] FIG. 2 is a photograph showing a state immediately after applying the composition of the present disclosure to the arm and showing that the state of the composition instantly changes to a gel state due to the temperature of the arm after application.
[FIG. 3] FIG. 3 is a photograph showing a state where cold water is applied to the composition of the present disclosure having changed to the gel state in FIG. 2, and showing that the state of the composition changed to the gel state is reversely changed to the sol state by the cold water (temperature lowering) and that the composition is removed by the cold water.

### Description of Embodiments

### (I) Composition

A composition of the present disclosure comprises (A) a polymer compound which has a sol-gel transition temperature and reversibly becomes a liquid state (sol state) at a temperature lower than the sol-gel transition temperature and (B) an oligopeptide having a cell proliferation function.

The composition of the present disclosure may be in the form of a solution comprising the above polymer compound (A) and the above oligopeptide (B). Such a solution can be obtained by mixing a solution of the above polymer compound (e.g., an aqueous solution) and a solution of an oligopeptide having a cell proliferation function (e.g., an aqueous solution). The composition of the present disclosure may also be in the form of a laminate comprising the above polymer compound (A) and the above oligopeptide (B) in separate layers, for example. Such a laminate can be obtained by a method such as forming the above polymer compound (A) and the above oligopeptide (B) as separate layers and laminating them appropriately (e.g., forming a layer comprising one component and applying a solution comprising the other to the layer or laminating sheets or the like).

In one embodiment, the composition of the present disclosure may be a solution mixed with water or a water-soluble organic solvent. However, even in a case where the composition of the present disclosure is in the state of a solution mixed with water or a water-soluble organic solvent, the state of the composition of the present disclosure have to reversibly change between a sol state and a gel state at the transition temperature of the polymer compound.

In one aspect, the aqueous organic solvent includes, for example, alcohols such as ethyl alcohol, polyhydric alcohols such as ethylene glycol, diethylene glycol, dipropylene glycol, glycerin, and 1,3-butylene glycol, and a polar organic solvent such as dimethylformamide and dimethylsulfoxide. Each of them may be used alone or two or more kinds of them may be combined. A preferable mixed solvent is a mixed solvent of water, propylene glycol, and ethyl alcohol.

The composition of the present disclosure may optionally comprise an active substance. In the present disclosure, "active substance" refers to a physiologically active substance other than the essential components (A) and (B) of the above composition. In one embodiment, the active substance in the present disclosure is a bactericide and the like. Examples of bactericides include, for example, benzalkonium chloride, cetylpyridinium chloride, and alcohols (ethanol, isopropanol, etc.) but are not limited to them. In one embodiment, the active substance is, for example, glycyrrhizinic acid, isopropylmethylphenol, hinokithiol, a Swertia japonica extract, Capsicum tincture, or vitamins (e.g., vitamin A, vitamin B, vitamin C, vitamin D, vitamin E, vitamin F, Vitamin H, Vitamin K, Vitamin P, Vitamin U, pantothenyl alcohol, carcinin, ferulic acid, γ-orizanol, lipoic acid, ollotic acid, or derivatives thereof). The active substance can also include a local anesthetic such as dibucaine, tetracaine, and lidocaine, a pain reliever such as a non-steroidal or steroidal anti-inflammatory agent, and a preservative such as an antioxidant and an antiseptic. As these active substances, conventionally and publicly known ones can be used. The composition of the present disclosure comprising the above active substances leads to the promotion of use for a coating agent, various substances, and the like.

In the present disclosure, the composition comprises an active substance in an amount in a range of 0.005% to 10% by mass, preferably 0.01% to 2.0% by mass.

In addition to the above components, commonly-used additives such as a fragrance, a colorant, a pH adjuster, a surfactant, and a propellant can be added to the composition of the present disclosure as required. The amount of each of the additives is preferably in a range of 0.001% to 5% by mass, preferably 0.01% to 2.0% by mass.

The composition of the present disclosure has a sol-gel transition temperature in a range of 20°C to 45°C, preferably 20°C to 40°C, more preferably 20°C to 37°C, still more preferably 20°C to 30°C, similarly to a polymer compound described later.

The component (A) and component (B) of the composition of the present disclosure will be described in detail below. In the present disclosure, the following description is illustrative of the present disclosure and the present disclosure is not limited to the following description.

### (A) Polymer compound

The polymer compound in the present disclosure has a hydrophobic portion and a hydrophilic portion in a molecule. The hydrophilic portion of the polymer compound is required for the polymer compound to be water-soluble at a temperature lower than the sol-gel transition temperature described above. Additionally, the hydrophobic portion is required for the polymer to transform to a gel state at a temperature higher than the sol-gel transition temperature. In other words, a bond between the hydrophobic portions is needed to form a crosslinking point in a gel.

The polymer compound of the present disclosure has a sol-gel transition temperature in a range of 20°C to 45°C, preferably 20°C to 40°C, more preferably 20°C to 37°C, still more preferably 20°C to 30°C.

The polymer compound of the present disclosure utilizes the properties described below of the bond between the hydrophobic portions in the polymer compound, that is, a hydrophobic bond.

The hydrophobic bond has a property that its binding strength increases as the temperature rises, so that the strength of a cross-link and a crosslinking density increase as the temperature rises, and in the present disclosure, the state can be changed to a gelled state at a temperature higher than the sol-gel transition temperature. Further, due to a property that the temperature dependence of the hydrophobic bind is reversible, the polymer compound of the present disclosure causes the sol-gel transition reversibly.

On the other hand, the hydrophilic portion in the polymer compound described above is necessary so that the polymer compound transforms to a sol state at a temperature lower than the sol-gel transition temperature. The hydrophilic portion is also necessary for forming gel while preventing the polymer compound from coagulating due to the hydrophobic bind strengthened too much at a temperature higher than the sol-gel transition temperature.

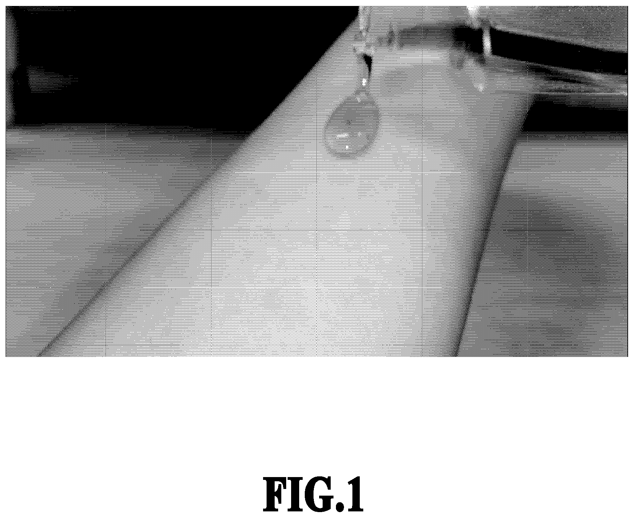

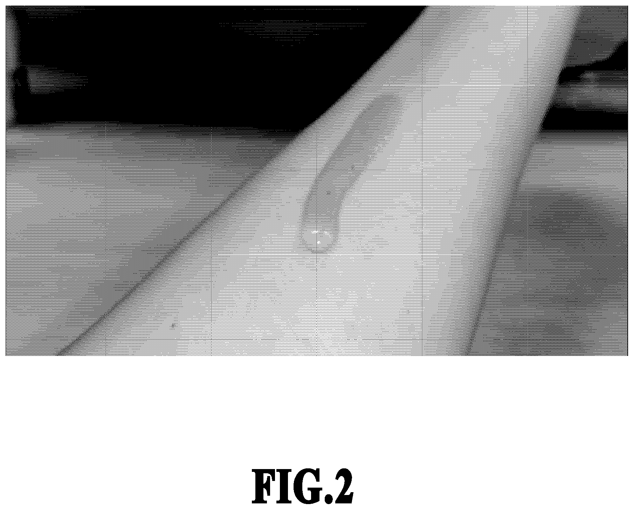

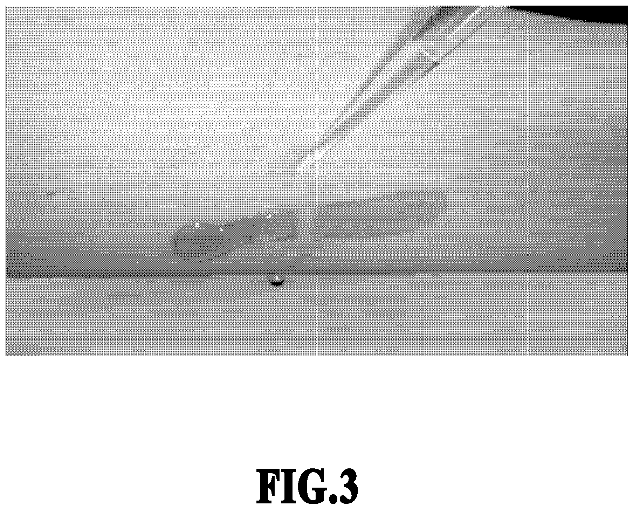

In a case where the polymer compound of the present disclosure is applied to a substrate, the state of the polymer compound transforms from a sol state to a gel state at a predetermined temperature or higher. For example, FIGs. 1 to 3 show changes in a state where a solution comprising a polymer compound is applied to human skin. When a solution (polymer compound in sol state) comprising the polymer compound of the present disclosure is applied to human skin (e.g. an arm) (see FIG. 1), a sol state is immediately transformed to a gel state due to a skin temperature (about 36°C) (see Fig. 2). Then, when cold water (e.g. 0°C) is poured to the polymer compound in gel state on the skin, the polymer compound transforms from the gel state to the sol state due to the temperature of the cold water, and the gel can be washed away with the cold water (dissolve in the cold water) (see Fig. 3).

### Definition

### (1) Sol-gel transition temperature

In the present disclosure, "sol state," "gel state," and "sol-gel transition temperature" are defined as follows. For the definition, see a literature (Polymer Journal, 18 (5), 411-416 (1986)).

1 mL of a polymer solution (a polymer solution comprising a polymer compound shown below) is poured into a test tube having an inner diameter of 1 cm and held in a water bath at a predetermined temperature (constant temperature) for 12 hours. In a case where a solution/air interface (meniscus) deforms under the weight of the solution (including a case where the solution flows out) when the test tube is turned upside down afterwards, the polymer solution is defined as being in the "sol state" at the above predetermined temperature. On the other hand, in a case where the solution/air interface (meniscus) described above does not deform under the weight of the solution even when the test tube is turned upside down, the solution is defined as being in a "gel state" at the above predetermined temperature.

In a case where the "predetermined temperature" described above is gradually increased (e.g. in increments of 1°C) to determine a temperature at which the "sol state" is transformed to the "gel state," the transition temperature determined by this is defined as a "sol-gel transition temperature." At this time, the "predetermined temperature" may be decreased in increments of, for example, 1°C to determine the temperature at which the "gel state" is transformed to the "sol state."

The polymer compound comprising the hydrophobic portion and the hydrophilic portion of the present disclosure is, for example, a polyalkylene oxide block copolymer represented by a block copolymer of polypropylene oxide and polyethylene oxide; etherified cellulose such as methyl cellulose and hydroxypropyl cellulose; a chitosan derivative (K. R. Holme, et al., Macromolecules, 24,3828 (1991)); modified polysaccharides such as a pullulan derivative (Shigeru Deguchi, et al., Polymer Preprints, Japan, 19,936 (1990)); a conjugate of a temperature-sensitive polymer and a water-soluble polymer compound represented, for example, by a poly N-substituted (meth)acrylamide derivative, partially acetylated polyvinyl alcohol, and polyvinyl methyl ether (e.g. Takehisa Matsuda, et al., Polymer Preprints, Japan, 39 (8), 2559 (1990)), but are not limited thereto.

The polymer compound of the present disclosure can also be prepared by cross-linking the polymer compound comprising the hydrophobic portion and hydrophilic portion described above with a cross-linking agent.

The sol-gel transition temperature of the polymer compound of the present disclosure can be adjusted based on the structure of the hydrophobic portion and the hydrophilic portion in the polymer compound, the hydrophobicity or hydrophilicity of the polymer compound, a molecular weight, and the like. The sol-gel transition temperature of the polymer compound can also be adjusted appropriately by adjusting the degree of cross-linking of the polymer compound. For the polymer compound of the present disclosure, although there is no limitation on the adjustment of the sol-gel transition temperature, it is preferable to appropriately adjust the sol-gel transition temperature of the polymer compound within the range of 0°C to 60° C.

The concentration of the polymer compound in the composition of the present disclosure depends on a sol-gel transition temperature to be set and the like. In a case where the polymer compound is used in a mixture with a predetermined oligopeptide as described later, the concentration is 0.1% to 30% by mass, preferably 1% to 20% by mass, based on the total mass of the composition.

### (B) Oligopeptide having a cell proliferation function

The composition of the present disclosure comprises an oligopeptide having a cell proliferation function. The oligopeptide is an oligopeptide having 3 to 7 amino acid units comprising a prolyl-isoleucyl-glycyl unit or an isoleucyl-glycyl-serine unit, and a salt thereof.

The oligopeptide of the present disclosure has only to have the function of promoting the proliferation of a desired cell. In one embodiment, the oligopeptide of the present disclosure preferably has the function of promoting the proliferation of an epithelial cell in particular. In another embodiment, the oligopeptide of the present disclosure preferably has the function of promoting cell regeneration during an epithelial graft, a skin ulcer, and aged skin restoration, in addition to the above function of promoting the proliferation of an epithelial cell. In still another embodiment, the oligopeptide of the present disclosure preferably has the function of proliferating a cell such as an iPS cell and an ES cell.

As long as the oligopeptide of the present disclosure has a desired cell proliferation function, the oligopeptide of the present disclosure may be not only the oligopeptide itself having the above-described number of amino acids but also a polypeptide having these oligopeptide units as constituent units in the molecule. In one embodiment, the oligopeptide of the present disclosure is preferably water-soluble.

In one embodiment, an oligopeptide having a specific amino acid unit exhibits an excellent effect of promoting the proliferation of an epithelial cell. Such an oligopeptide having the function of proliferating an epithelial cell is suitable for various uses described later and thus are specifically preferable. Some peptides having the function of promoting the proliferation of an epithelial cell are disclosed, for example, in Japanese Patent No. 4654418 (Patent Literature 3) and specifically include the following.

As described above, the oligopeptide of the present disclosure is preferably a peptide having 3 to 7 amino acids. For example, a tripeptide includes isoleucyl-glycyl-serine and prolyl-isoleucyl-glycine. Further, a tetrapeptide includes one having an amino acid residue such as a glycyl, alanyl, arginyl, asparagyl, ridill, ceryl, noryl, or dartamil group bonded to the front or rear of the above tripeptide. Glycyl-prolyl-isoleucyl-glycine (SEQ ID NO: 1) and prolyl-isoleucyl-glycyl-serine (SEQ ID NO: 2) are preferred.

A pentapeptide includes, for example, one having an amino acid residue such as a ceryl group or a threonyl group bonded to the front or rear of a glycyl-prolyl-isoleucyl-glycyl (SEQ ID NO: 1) group. Glycy-prolyl-isoleucyl-glycyl-serine (SEQ ID NO: 3) and glycyl-prolyl-isoleucyl-glycyl-threonine (SEQ ID NO: 4) are preferred peptides.

A hexapeptide and a heptapeptide includes ones having the above pentapeptide unit at a carboxyl terminal group. For example, alanyl-glycyl-prolyl-isoleucyl-glycyl-serine (SEQ ID NO: 5) and seryl-glycyl-prolyl-isoleucyl-glycyl-serine (SEQ ID NO: 6) are preferred.

The oligopeptide of the present disclosure may be in a free form or may be a salt thereof. This salt is, for example, a sodium salt, a potassium salt, a lithium salt, or an ammonium salt.

The oligopeptide of the present disclosure can be produced by reacting a raw material amino acid having an protected α-amino group with an amino acid having a protected carboxyl group by a method commonly used for forming a peptide bond in polypeptide synthesis, such as, a condensing agent method, an active ester method, an azide method, and a mixed acid anhydride method to form a peptide, and removing the protecting group; and then the steps are repeated.

The condensing agent method is the most common method for forming a peptide bond. A condensing agent used in this method includes, for example, dicyclohexylcarbodiimide (DCC), diisopropylcarbodiimide (DIPC), N-ethyl-N'-3-dimethylaminopropylcarbodiimide (WSCI) and its hydrochloride (WSCI, HCl), benzotriazole-1-yloxy-trisdimethylaminophosphonium hexafluorophosphate (BOP), or diphenylphosphoryldiazide (DPPA), which are used alone, or in combination with N-hydroxysuccinimide (HONSu), 1-hydroxybenzotriazole (HOBt), or 3-hydroxy-4-oxo-3,4-dihydro-1,2,3-benzotriazine (HOObt).

An active ester in the active ester method includes, for example, p-nitrophenyl ester (ONp), N-hydroxysuccinimide ester (ONSu), and pentafluorophenyl ester (OPfp).

The azide method is a method in which an amino acid or a peptide is reacted with anhydrous hydrazine to form a corresponding hydrazide. This method is known as a segment condensation method with low racemization.

Further, the mixed acid anhydride method is a method in which isobutyloxycarbonyl chloride, diethyl acetyl chloride, trimethyl acetyl chloride, and the like are used to form a mixed anhydride at a carboxyl group of an amino acid. This method has an advantage that the carboxyl group can be remarkably activated at low temperatures.

In one aspect, protecting groups easily eliminated by deprotection such as acid treatment, hydrolysis, or catalytic reduction are used to protect an amino acid. In one embodiment, a protecting group for an α-amino group includes a benzyloxycarbonyl group, a tert-butoxycarbonyl group, a 9-fluorenylmethoxycarbonyl group, a 3-nitro-2-pyridinesulfenyl group, and a methoxybenzyloxycarbonyl group. In one embodiment, a protection group for a carboxyl group includes methyl or ethyl ester, benzyl ester, tert-butyl ester, and phenacyl ester.

In the case of an α-amino acid having a hydroxyl group in a side chain, this hydroxyl group have to be protected. In one embodiment, the protecting group is a benzyl group or a tert-butyl group. A benzyl group can be easily eliminated by catalytic reduction with a platinum black catalyst or by treatment with strong acid. The tert-butyl group can be easily eliminated by treatment with weak acid.

The α-amino acid ester and the amino acid with the amino group or hydroxyl group protected as described above can be easily obtained as a commercial product.

A method of producing the oligopeptide of the present disclosure may be a liquid phase method in which raw material amino acids or derivatives thereof are homogeneously dissolved in a solvent to react them, or a solid phase method in which the raw material amino acids or the derivatives thereof are condensed on an insoluble resin to elongate a peptide chain. In one embodiment, the oligopeptide of the present disclosure is preferably produced with an automatic solid-phase synthesizer. According to this method, a desired oligopeptide can be produced in a short time and at a high purity.

The oligopeptide or salt thereof used in the composition of the present disclosure is obtained as a racemate. Such a racemate can also be used as it is, but if desired, the racemate can be optically resolved by any conventional method for obtaining an optical active substance. An optical resolution method includes a method in which a diastereomer is formed from a racemate of amino acid and an appropriate optically active substance to subject the diastereomer to fractional crystallization, a method using an enzyme, or a method using high-performance liquid chromatography (HPLC) using a chiral carrier.

The oligopeptide of the present disclosure is soluble in water or alcohols. The oligopeptide of the present disclosure can be identified by a conventionally-used means such as mass spectrometry, an infrared absorption spectrum, or high-performance liquid chromatography.

The oligopeptide of the present disclosure has the function of directly promoting epidermis cell proliferation in addition to the function of directly promoting epithelial cell proliferation. That is, the oligopeptide of the present disclosure is useful in a cultured skin graft or in the treatment of skin ulcer, skin defective injury, or the like.

The oligopeptide of the present disclosure may be contained in a concentration of 0.0001% to 10% by mass in the composition of the present disclosure.

### (II) Use of the composition

The composition of the present disclosure is used for various purposes. In one embodiment, the composition can be used for a coating agent, proliferation of various cells, preservation of living tissues, and the like. Specific examples of the use of the composition of the present disclosure will be described below. The present disclosure is not limited to the following use.

### (a) Coating agent

The composition of the present disclosure can be used as a coating agent for coating various substrates. For example, a coating agent comprising the composition of the present disclosure may be provided. In one embodiment, the composition of the present disclosure may be provided as a coating agent in a liquid state (sol state). Such a coating agent may be provided in the form of a spray agent, a liniment, an immersion agent, or the like. In the case of the spray agent, the coating agent can be obtained by mixing the composition of the present disclosure with an appropriate propellant and/or solvent to fill a sprayer. In the case of a coating agent and an immersion agent, the composition of the present disclosure may be dissolved in an appropriate solvent (provided that a sol-gel transition occurs). The coating agents in these formulations can be applied to an affected portion through conventional procedures.

In one embodiment, the composition of the present disclosure can be used as a coating agent to coat a burn, a wound, or the like. For example, the composition of the present disclosure can be used as an alternative to conventional coating materials such as an adhesive plaster and a bandage. When the composition of the present disclosure is applied to a portion (hereinafter, also referred to as an affected portion) such as a burn or a wound, the state of the composition changes from a liquid state (sol state) to a solid state (gel state) due to the temperature of the affected portion (for example, a human skin surface), so that the affected portion can be easily protected. For example, as shown in FIGs. 1 to 3, the state of the polymer compound used in the present disclosure can change from the sol state to the gel state due to a human body temperature or the temperature of the affected portion having a temperature which has increased due to a burn, a wound, or the like. Thus, the state of the composition of the present disclosure containing the polymer compound can also rapidly change from the sol state to the gel state due to the human body temperature or the temperature of the affected portion. For example, in the case of a burn, a wound, or the like, rapidly coating the affected portion with the composition of the present disclosure can rapidly block the affected portion from oxygen in the air and promote healing of the burn, wound, or the like and skin regeneration.

For example, in one embodiment of the present disclosure in which the composition of the present disclosure comprising the above polymer compound and oligopeptide having the epithelial cell proliferation function is used to coat an affected portion such as a burn with the composition of the present disclosure, the composition and the coating agent of the present disclosure can prevent a proliferation of fibroblasts such as keloids and enhance the effect of regenerating the affected portion by the epithelial cell proliferation function (regeneration of epidermis) on the affected portion. In this embodiment, since the oligopeptide selectively exhibits a proliferation function on an epithelial cell, a coating agent of a composition comprising the oligopeptide has a low possibility of growing other cells. Thus, a coating agent comprising the composition of the present disclosure is extremely useful.

Without wishing to be bound by any theory, this function is thought to be caused for the following reasons. Scaffolding is necessary for the proliferation of fibroblasts, and the polymer compound of the present disclosure has more space than space enough to be the scaffolding. Thus, it is considered that the fibroblasts cannot proliferate in the composition of the present disclosure or the affected portion. Further, the state of the coating agent of the present disclosure reversibly changes to a liquid state at a temperature lower than the transition temperature as a coating agent for coating a burn, a wound, or the like, so that the coating agent can be removed by a convenient method such as washing with water so as not to put a load on an affected portion such as a burn or a wound.

The coating agent of this embodiment may further comprise an active substance that promotes healing of a burn, a wound, or the like. Such an active substance includes cell proliferation/differentiation factors and cell adhesion factors.

The cell proliferation/differentiation factors include a platelet-derived growth factor (PDGF), an epidermal growth factor (EGF), interleukins, and the like. It is preferable to use about 10⁻⁷ to 10⁻³ parts, preferably about 10⁻⁶ to 10⁻⁴ parts of these cell proliferation/differentiation factors with respect to 100 parts of the polymer in the composition of the present disclosure.

The cell adhesion factors include an extracellular matrix such as collagen, fibronectin, vitronectin, laminin, proteoglycan and glycosaminoglycan; and gelatin. About 0.01 to 50 parts, preferably about 0.1 to 10 parts of these cell adhesion factors can be used with respect to 100 parts of the polymer in the composition of the present disclosure.

The coating agent of the present disclosure containing the cell-proliferating active substance as described above can promote healing of an affected portion such as a burn or a wound and can accelerate healing of the burn or wound.

The application frequency of the coating agent of the present disclosure is not specifically limited, but for example, the coating agent of the present disclosure can be applied to the affected portion about one to five times a day.

### (b) Cell-proliferating substrate and a substrate for preserving or transporting a tissue or a tissue piece (e.g. an organ for transplantation)

The composition of the present disclosure can be used for a cell-proliferating substrate, a substrate for preserving or transporting a tissue or a tissue piece (e.g., an organ for transplantation), or the like. For example, the cell-proliferating substrate includes a three-dimensional culture substrate for a cell such as an iPS cell and an ES cell.

A preferred example of a culture substrate using the composition of the present disclosure will be described below.

First, the composition of the present disclosure is uniformly dissolved in a desired medium necessary for cell proliferation at a temperature lower than the sol-gel transition temperature of the composition. At this time, it is also possible to dissolve an active substance necessary for cell proliferation at the same time as appropriate.

Next, desired cells are mixed and dispersed in the medium solution comprising the composition of the present disclosure. Then, increasing the temperature of the mixture to a temperature higher than the sol-gel transition temperature allows the mixture to promptly and easily become gel.

Methods of forming a culture substrate containing a cell intended to be proliferated include, for example, the following methods. These forming methods are examples and the present disclosure is not limited to these methods.

Methods of preparing the culture substrate containing cells into particles include, for example, the following methods:
(1) a method wherein the medium solution including the composition of the present disclosure in which cells and/or a cell mass is mixed and dispersed at a temperature lower than the sol-gel transition temperature is added into a medium warmed above the sol-gel transition temperature thereby to gelate the solution; or
(2) a method wherein the medium solution comprising the composition of the present disclosure in which cells and/or a cell mass is mixed and dispersed is added to a large amount of liquid paraffin at a temperature lower than the sol-gel transition temperature and suspended under stirring, and then a temperature of the suspension is elevated at a temperature higher than the sol-gel transition temperature thereby to gelate the suspended particles, and then a medium warmed above the sol-gel transition temperature is added to the suspension of the gelated particle, and a resulting suspension is centrifuged.

In method (2) above, the centrifugation can separate a fluid paraffin layer as upper layer and a medium suspension layer of the gel particles as a lower layer, and easily separate the cells and/or the cell mass-containing gel particles from the liquid paraffin.

To prepare a cell-containing culture substrate into a fiber, the medium solution of the composition of the present disclosure may be continuously extruded into a medium warmed above the sol-gel transition temperature from a microdispenser or the like.

To prepare the cell-containing culture substrate into a film or sheet, the medium solution of the composition of the present disclosure may be spread onto a planar support warmed above the sol-gel transition temperature and be gelled.

Here, in order to recover or subculture the cells and/or cell mass cultured in the culture substrate of the present disclosure, it is necessary to separate the cells from the substrate. The simplest and easiest method as the separation method is to, first, lower the temperature of the culture substrate to a temperature lower than the sol-gel transition temperature of the culture substrate (the sol-gel transition temperature of the composition of the present disclosure) and bring the culture substrate to the sol state to prepare a suspension solution of the cells and/or cell mass. This method has an advantage that cultured cells and/or a cancer cell mass can be separated from the culture substrate without being damaged.

In one embodiment of the present disclosure, substrates for preserving or transporting a tissue or a tissue piece include a substrate for, in transporting an organ, a graft, or the like taken out for organ transplantation or skin graft in a predetermined container, filling space between the container and the tissue or the graft.

Such a substrate for preservation or transportation can be prepared by cooling the composition of the present disclosure to a predetermined temperature or lower and pouring it in a sol state into a container accommodating a desired tissue or a tissue piece. In order to recover a tissue or a tissue piece from the substrate, the temperature of a substrate for preservation or transportation may be lowered to a temperature lower than the sol-gel transition temperature of the substrate (the sol-gel transition temperature of the composition of the present disclosure) to bring the substrate to a sol state and wash it away with cooled purified water or the like.

The present disclosure includes a method of treating an affected portion comprising a burn or a wound using the above composition or a coating agent comprising the composition. The method comprises:
step a) applying a composition or a coating agent comprising the composition to an affected portion comprising a burn or a wound, the composition comprising:
(A) a polymer compound which has a sol-gel transition temperature, reversibly transforms to a sol state at a temperature lower than the transition temperature, and comprises hydrophobic and hydrophilic portions; and
(B) an oligopeptide having a cell proliferation function,
   wherein the composition or the coating agent causes a sol-gel transition on the affected portion and transforms to the sol state thereby to coat the affected portion.

In one embodiment of step a), the composition or the coating agent comprising the composition of the present disclosure is applied onto a burn or a wound in a liquid state (sol state). In the methods of treating a burn or a wound of the present disclosure, the composition of the present disclosure itself can be applied to an affected portion, or a coating agent comprising the composition of the present disclosure can also be applied to the affected portion. As the coating agent, those described in the above coating agent section can be used. The composition and coating agent of the present disclosure to be used may be provided in the form of a spray agent, a liniment, an immersion agent, or the like. A spray agent can be obtained by mixing the composition of the present disclosure with an appropriate propellant and/or solvent to fill a sprayer. In the case of a liniment and an immersion agent, the composition of the present disclosure may be dissolved in an appropriate solvent (provided that the sol-gel transition occurs). The compositions of the present disclosure in these formulations can be applied to the affected portion through conventional procedures such as spraying, application with a blade, and immersion in a solvent comprising the composition of the present disclosure. A coating agent comprising the composition of the present disclosure can also be applied in the same manner as described above.

In one embodiment, in the method of treating a burn or a wound of the present disclosure, the composition or coating agent of the present disclosure can be used as an alternative to conventional coating materials such as an adhesive plaster and a bandage. In the method of treating a burn or a wound of the present disclosure, when the composition or coating agent of the present disclosure is applied to a portion (affected portion) such as a burn or a wound, the composition or coating agent of the present disclosure transforms from a liquid state (sol state) to a solid state (gel state) due to the temperature of the affected portion (e.g., a human skin surface), so that the affected portion can be easily protected. For example, as shown in FIGs. 1 to 3, the state of the polymer compound used in the treatment method in the present disclosure can transform from the sol state to the gel state due to a human body temperature. Thus, the state of the composition of the present disclosure containing the polymer compound can also rapidly change from the sol state to the gel state on the affected portion. For example, in the case of a burn, rapidly coating the affected portion with the composition or coating agent comprising the composition of the present disclosure can rapidly block the affected portion from oxygen in the air and promote healing of the burn and skin regeneration.

In a method of treating a burn or a wound using the composition (for example, one comprising the above polymer compound and oligopeptide having an epithelial cell proliferation function) or a coating agent comprising the composition of the present disclosure of one embodiment, the composition or the coating agent comprising the composition of the present disclosure coats an affected portion such as a burn or a wound, so that proliferation of fibroblasts such as keloids can be prevented, and a regeneration effect on the affected portion can be enhanced by an epithelial cell proliferation function (regeneration of epidermis) on the affected portion. In this embodiment, since the oligopeptide has a selective proliferation function on an epithelial cell, a treatment method of the present disclosure using the composition comprising the oligopeptide or the coating agent comprising the composition is advantageous without the possibility of proliferation of other cells.

The composition of the present disclosure used in the treatment method of the present disclosure may optionally contain an active substance. In the present disclosure, "active substance" refers to a physiologically active substance other than the essential components (A) and (B) of the above composition. In one embodiment, the active substance in the present disclosure is a bactericide or the like. Examples of bactericides include, but are not limited to, benzalkonium chloride, cetylpyridinium chloride, and alcohols (ethanol, isopropanol, etc.). In one embodiment, the active substance includes, for example, glycyrrhizinic acid, isopropylmethylphenol, hinokithiol, a Swertia japonica extract, Capsicum tincture, and vitamins (e.g., vitamin A, vitamin B, vitamin C, vitamin D, vitamin E, vitamin F, vitamin H, vitamin K, vitamin P, vitamin U, pantothenyl alcohol, carnitine, ferulic acid, γ-oryzanol, lipoic acid, orotic acid, or derivatives thereof). The active substance can also include a local anesthetic such as dibucaine, tetracaine, and lidocaine, a pain reliever such as a non-steroidal or steroidal anti-inflammatory agent, and a preservative such as an antioxidant and an antiseptic. As these active substances, conventionally and publicly known ones can be used. In the treatment method of the present disclosure, the treatment effect can be further promoted by comprising these active agents in the composition of the present disclosure.

The method of treating an affected portion comprising a burn or a wound can further comprise, following step a) above, a step (step b)) of cooling the composition or the coating agent comprising the composition of the present disclosure to remove the composition or the coating agent comprising the composition from the affected portion.

In one embodiment, step b) can be performed by cooling the applied composition or coating agent comprising the composition of the present disclosure to a temperature at which the state of the composition or the coating agent changes to a liquid state (sol state) to wash away the composition or the coating agent comprising the composition of the present disclosure with cooled purified water or the like.

In the treatment method of the present disclosure, the oligopeptide included in the composition or the coating agent comprising the composition of the present disclosure can remain in the affected portion after the wash in step b). Thus, a treatment effect can be sustained and/or promoted in the treatment of a burn or a wound after the composition or the coating agent comprising the composition of the present disclosure has been washed away.

### (Examples)

The present disclosure will be described in more detail in the following examples. The scope of the present disclosure is not limited by the following examples but is defined by the claims.

### (Preparation Example 1)

In this example, a polymer compound (F-127 polymer) was used. This polymer compound is disclosed in Japanese Patent No. 3190145 (Patent Literature 1) or Japanese Patent No. 3190147 (Patent Literature 2) and was synthesized and sterilized according to a method disclosed in these literatures.

### (Synthesis of F-127 Polymer)

The F-127 polymer was obtained in accordance with the above patent literatures by reacting purlonic F-127 (produced by Asahi Denka Co., Ltd.) (registered trademark) which is a block copolymer of polypropylene oxide and polyethylene oxide, with hexamethylene diisocyanate in the presence of phosphorus pentoxide.

As the obtained F-127 polymer was dissolved in distilled water at a concentration of 10 wt% under ice-cooling and slowly warmed, a viscosity gradually increased from 21°C, and the polymer solution was solidified at about 27°C and became hydrogel. When the hydrogel was cooled, it was returned to an aqueous solution at 21°C. This change was reversibly observed repeatedly.

### (Method of Sterilizing F-127 Polymer)

The F-127 polymer obtained as described above was sterilized through a procedure disclosed in Japanese Patent No. 3190145 (Patent Literature 1) above. The obtained F-127 polymer was confirmed to have been completely sterilized.

Next, the oligopeptide of the present disclosure was prepared. This preparation follows a procedure disclosed in International Publication No. WO 2005/095441.

### (Preparation Example 2)

Racemic isoleucyl-glycyl-serine was obtained according to the procedure disclosed in International Publication No. WO 2005/095441 above. Its physical properties and the like were the same as those described in the publication.

### (Preparation Example 3)

Racemic prolyl-isoleucyl-glycine was obtained according to the procedure disclosed in International Publication No. WO 2005/095441 above. Its physical properties and the like were the same as those described in the publication.

### (Preparation Example 4)

Racemic prolyl-isoleucyl-glycyl-serine (SEQ ID NO: 2) was obtained according to the procedure disclosed in International Publication No. WO 2005/095441 above. Its physical properties and the like were the same as those described in the publication.

### (Preparation Example 5)

Racemic glycyl-prolyl-isoleucyl-glycine (SEQ ID NO: 1) was obtained according to the procedure disclosed in International Publication No. WO 2005/095441 above. Its physical properties and the like were the same as those described in the publication.

### (Preparation Example 6)

Racemic glycyl-prolyl-isoleucyl-glycyl-serine (SEQ ID NO: 3) was obtained according to the procedure disclosed in International Publication No. WO 2005/095441 above. Its physical properties and the like were the same as those described in the publication.

### (Preparation Example 7)

Racemic glycyl-prolyl-isoleucyl-glycyl-threonine (SEQ ID NO: 4) was obtained according to the procedure disclosed in International Publication No. WO 2005/095441 above. Its physical properties and the like were the same as those described in the publication.

### (Example 1)

The composition of the present disclosure will be described in detail below.

The polymer compound (F-127 polymer) (15 mg) obtained in Preparation Example 1 above and an oligopeptide (428 µg) obtained in Preparation Examples 2 to 7 were each dissolved in 150 µL of water and mixed. An obtained composition had the same sol-gel transition temperature as that of the above polymer compound.

### (Example 2)

The following shows an example of an ES-cell-culturing substrate using the composition of the present disclosure.

Mouse-derived ES cells were dissolved in warm water at 37°C and suspended in D-MEM (Wako Pure Chemical Industries, Ltd., containing 10% of Gibco FBS). This was seeded in a polymer compound (containing 1 µM of tripeptide) dissolved while being cooled at 4°C overnight and was gelled at 37°C for 30 minutes in an atmosphere containing 5% CO₂. Then, culture media were layered and cultured at 37°C in an atmosphere containing 5% CO₂.

During subculture, a culture was cooled to 4°C, solated to recover cells, and centrifuged at 1,000 rpm for one minute. After centrifugation, supernatant was removed, and a cell suspension to which a culture solution was added was seeded in a cooled and dissolved polymer compound (containing tripeptide) and gelled at 37°C for 30 minutes in an atmosphere containing 5% CO₂. After gelation, culture media were layered and cultured at 37°C in an atmosphere containing 5% CO₂.

In this example, it was found that an ES cell can be cultured with a tripeptide-containing polymer compound.

An ES cell is commonly cultured on a feeder cell, but can be cultured without feeder cell. Thus, culture in the present disclosure does not require cell-peeling and separation from a feeder cell during subculture and recovery. The ES cell can be recovered only by cooling.

### (Example 3)

The following shows an example of cell proliferation using the polymer compound and oligopeptide (corresponding to the composition of the present disclosure) of the present disclosure.

Test cell:
human keratinocyte cell (KAC Co., Ltd.: KER110)

Culturing substrate:
the composition of the present disclosure comprising the polymer compound (TGP in Preparation Example 1) and oligopeptide of the present disclosure.

### 1. Culture of epithelial cells

Frozen human keratinocyte cells (KAC Co., Ltd.: KER110) were dissolved in warm water at 37°C, suspended in EpiLife Medium (Thermo Fisher Scientific, Inc.: MEPI500CA) to which Human Keratinocyte Growth Supplement (HKGS) (Thermo Fisher Scientific, Inc.: S0015) was added, seeded in a 25 cm² flask, and cultured at 37°C in an atmosphere containing 5% CO₂.

### 2. Subculture of epithelial cells

The cultured cells were washed with DPBS (Thermo Fisher Scientific, Inc.: 14190144), and a trypsin solution (Thermo Fisher Scientific, Inc.: 12604013) was added to the cultured cells to recover cells and was centrifuged at 1,000 rpm for three minutes. After the centrifugation, supernatant was removed. A cell suspension obtained by adding a culture solution to a cell sediment was seeded in a 25 cm² flask and subcultured at 37°C in an atmosphere containing 5% CO₂.

### 3. Preparation of a culturing substrate and seeding to the culturing substrate

The tripeptide in Preparation Example 3 was dissolved in a culture medium to prepare six samples having concentrations of 1 µM, 3 µM, 10 µM, 30 µM, 100 µM, and 1000 µM. A control includes only a culture medium.

The polymer compound (TGP in Preparation Example 1) of the present disclosure is added into a 48-well plate and freeze-dried. 120 µL of the six samples of the above concentrations and the control culture medium were added to the respective wells, and each mixture was dissolved with cooling at 4°C overnight.

30 µL of a cell suspension recovered through the same procedure as the subculture operation was seeded in the polymer compound of the present disclosure in the 48-well plate and gelled at 37°C for 30 minutes in an atmosphere containing 5% CO₂. Then, 300 µL of the six samples of the above concentrations and control culture medium were layered and cultured at 37°C in an atmosphere containing 5% CO₂.

### 4. Cell proliferation test

After culturing for 7 days, 250 µL of culture supernatant was removed, 60 µL of Cell Counting Kit-8 (Doujin Kagaku Co., Ltd.) was added and the mixture was reacted overnight at 37°C in an atmosphere containing 5% CO₂. 80 µL of the supernatant of an obtained reaction product was dispensed into a 96-well plate. This supernatant was measured with a microplate reader at a measurement wavelength of 450 nm to evaluate cell proliferation.

### 5. Result

The cell proliferation rates of 1 µM, 3 µM, 10 µM, 30 µM, 100 µM, and 1000 µM samples were compared with that of a control, and the results are shown in Table 1 below.

### [Table 1]

**Table 1 Cell Proliferation Rates**

| Control | 100% |
|---|---|
| 1 µM | 114% |
| 3 µM | 116% |
| 10 µM | 118% |
| 30 µM | 126% |
| 100 µM | 126% |
| 1000 µM | 128% |

As a result of multiple comparison test of significance test Dunnett, a significance level of less than 1% (p < 0.01) was observed at all concentrations.

The present application includes the following disclosure:
(a1) A composition comprising:
   (A) a polymer compound which has a sol-gel transition temperature, reversibly transforms to a sol state at a temperature lower than the transition temperature, and comprises hydrophobic and hydrophilic portions; and
   (B) an oligopeptide having a cell proliferation function.
(a2) The composition according to (a1) above, wherein the oligopeptide having the cell proliferation function is a water-soluble oligopeptide having 3 to 7 amino acids comprising a prolyl-isoleucyl-glycyl unit or an isoleucyl-glycyl-serine unit and a water-soluble salt thereof.
(a3) The composition according to (a1) above, wherein the cell proliferation function of the oligopeptide is an epithelial cell proliferation function.
(a4) The composition according to (a1) above, wherein the polymer compound has a sol-gel transition temperature in a range of 20°C to 45°C.
(a5) The composition according to (a1) above, wherein the composition further comprises a bactericide, a local anesthetic, a pain reliever, and a preservative.
(a6) A coating agent comprising the composition according to (a1) above.
(a7) A cell-culturing substrate comprising the composition according to (a1) above.
(a8) The cell-culturing substrate according to (a7) above, the substrate being a three-dimensional culture substrate for an iPS cell or an ES cell.
(a9) A living-tissue-transporting substrate comprising the composition according to (a1) above.
(a10) Use of the composition according to (a1) above in cell culture.
(a11) The use according to (a10) above, wherein the cell culture is culture of an epithelial cell, a cancer cell, an iPS cell, or an ES cell.
(a12) A method of treating a burn or a wound, the method comprising:
   step a): applying a composition or a coating agent comprising the composition to an affected portion comprising a burn or a wound, the composition comprising:
   (A) a polymer compound which has a sol-gel transition temperature, reversibly transforms to a sol state at a temperature lower than the transition temperature, and comprises hydrophobic and hydrophilic portions; and
   (B) an oligopeptide having a cell proliferation function,
   wherein the composition or the coating agent causes a sol-gel transition on the affected portion and transforms to a sol state thereby to coat the affected portion.
(a13) The method of treating an affected portion comprising a burn or a wound according to (a12) above, the method further comprising, after step a) above, cooling the composition or the coating agent to remove the composition or the coating agent from the affected portion.
(a14) The method of treating a burn or a wound according to (a12) above, wherein the oligopeptide having the cell proliferation function is a water-soluble oligopeptide having 3 to 7 amino acids comprising a prolyl-isoleucyl-glycyl unit or an isoleucyl-glycyl-serine unit and a water-soluble salt thereof.
(a15) The method of treating a burn or a wound according to (a12) above, wherein the cell proliferation function of the oligopeptide is an epithelial cell proliferation function.
(a16) The method of treating a burn or a wound according to (a12) above, wherein the polymer compound has a sol-gel transition temperature in a range of 20°C to 45°C.
(a17) The method of treating a burn or a wound according to (a12) above, wherein the composition further comprises a bactericide, a local anesthetic, a pain reliever, and a preservative.

## Claims

1. A composition comprising:
(A) a polymer compound which has a sol-gel transition temperature, reversibly transforms a sol state at a temperature lower than the transition temperature, and comprises hydrophobic and hydrophilic portions; and
(B) an oligopeptide having a cell proliferation function.

2. The composition according to claim 1, wherein the oligopeptide having the cell proliferation function is a water-soluble oligopeptide having 3 to 7 amino acids comprising a prolyl-isoleucyl-glycyl unit or an isoleucyl-glycyl-serine unit and a water-soluble salt thereof.

3. The composition according to claim 1 or claim 2, wherein the cell proliferation function of the oligopeptide is an epithelial cell proliferation function.

4. The composition according to any one of claims 1 to 3, wherein the polymer compound has a sol-gel transition temperature in a range of 20°C to 45°C.

5. The composition according to any one of claims 1 to 4, further comprising a bactericide, a local anesthetic, a pain reliever, and a preservative.

6. A coating agent comprising the composition according to any one of claims 1 to 5.

7. A cell-culturing substrate comprising the composition according to any one of claims 1 to 5.

8. The cell-culturing substrate according to claim 7, the cell-culturing substrate being a three-dimensional culture substrate for an iPS cell or an ES cell.

9. A living-tissue-transporting substrate comprising the composition according to any one of claims 1 to 5.

10. Use of the composition according to any one of claims 1 to 5 in cell culture.

11. The use according to claim 10, wherein the cell culture is culture of an epithelial cell, a cancer cell, an iPS cell, or an ES cell.


### [Sequence Listing]

SEQUENCE LISTING

<110> International Frontier Technology Laboratory Co., Ltd.

<120>  A composition comprising thermosensitive gel and origopeptide, and
use the same

<130> IK-200

<150> JP2019-163166
<151> 2019-09-06

<160>  6

<170>  PatentIn version 3.1

<210>  1
<211>  4
<212>  PRT
<213>  Artificial

<220>
<223>  A synthesized oligopeptide

<220>
<221>  PEPTIDE
<222>  (1)..(4)
<223>  A partial structure of GPIGS peptide


<400>  1

Gly Pro Ile Gly
1


<210>  2
<211>  4
<212>  PRT
<213>  Artificial

<220>
<223>  A synthesized oligopeptide

<220>
<221>  PEPTIDE
<222>  (1)..(4)
<223>  A partial structure of GPIGS peptide


<400>  2

Pro Ile Gly Ser
1


<210>  3
<211>  5
<212>  PRT
<213>  Artificial

<220>
<223>  A synthesized oligopeptide

<220>
<221>  PEPTIDE
<222>  (1)..(5)
<223>

<400>  3

Gly Pro Ile Gly Ser
1               5


<210>  4
<211>  5
<212>  PRT
<213>  Artificial

<220>
<223>  A synthesized oligopeptide

<220>
<221>  PEPTIDE
<222>  (1)..(4)
<223>  The C-terminal Ser residue in GPIGS is replaced with Thr.


<400>  4

Gly Pro Ile Gly Thr
1               5


<210>  5
<211>  6
<212>  PRT
<213>  Artificial

<220>
<223>  A synthesized oligopeptide

<220>
<221>  PEPTIDE
<222>  (2)..(6)
<223>  A derivertive of a GPIGS peptide in which the N-terminal Gly resi
       due is modified with Ala.


<400>  5

Ala Gly Pro Ile Gly Ser
1               5


<210>  6
<211>  6
<212>  PRT
<213>  Artificial

<220>
<223>  A synthesized oligopeptide

<220>
<221>  PEPTIDE

<222>  (2)..(6)
<223>  A derivertive of a GPIGS peptide in which the N-terminal Gly resi
       due is modified with Ser.

<400>  6

Ser Gly Pro Ile Gly Ser
1               5